# EUROPEAN PATENT SPECIFICATION

(11) **EP 0 669 327 B1**
(45) Date of publication and mention of the grant of the patent: **06.10.1999**
(21) Application number: 95103953.6
(22) Date of filing: 12.04.1990
(51) Int. Cl.: C07D 281/10

(54) **Process for the preparation of 1,5-benzothiazepines by ring closure of amino esters**
Verfahren zur Herstellung von 1,5-Benzothiazepinen durch Ringschluss von Aminoestern
Procédé pour la préparation de 1,5-benzothiazepines par cyclisation d'esters aminés

(30) Priority: 13.04.1989 IT 2013489
(43) Date of publication of application: 30.08.1995
(62) Divisional of application: 90107048.2
(73) Proprietor: ZAMBON GROUP S.p.A., I-36100 Vicenza (IT)
(72) Inventor: Tentorio, Dario, I-22060 Vigano' (Como) (IT)

(56) References cited:
- EP-A- 0 343 474
- EP-A- 0 378 455
- EP-A- 0 395 302
- EP-A- 0 447 135
- CHEM. PHARM. BULL., vol.18, no.10, October 1970 pages 2028 - 2037

## Description

The present invention relates to a process for the preparation of 1,5-benzothiazepines and, more particularly, it relates to a process for the preparation of 1,5-benzothiazepines useful as intermediates in the synthesis of optically active 2-(4-methoxyphenyl)-1,5-benzothiazepin-4(5H)ones of formula 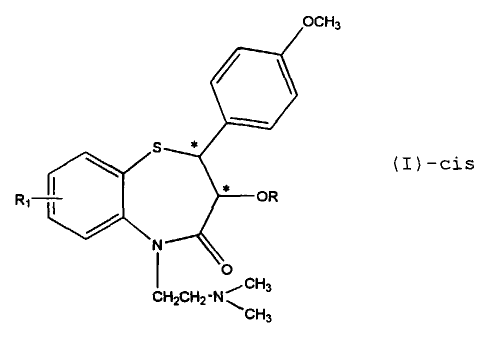 wherein
R represents a hydrogen atom or acetyl,
R₁ represents a hydrogen atom or chlorine atom,
   the asterisks mark the asymmetric carbon atoms.

Specific examples of the compounds of formula I are Diltiazem, (+)-(2S,3S)-3-acetoxy-5-[2-(dimethylamino)-ethyl]-2,3-dihydro-2-(4-methoxyphenyl)-1,5-benzothiazepin-4(5H)-one (Merck Index, X Ed., No. 3189, page 466) and TA-3090, (+)-(2S,3S)-3-acetoxy-8-chloro-5-[2-(dimethylamino)-ethyl]-2,3-dihydro-2-(4-methoxyphenyl)-1,5-benzothiazepin-4(5H)-one (Annual Drug Data Report 1987, page 507).

Various methods for the preparation of the compounds of formula I are known in the literature such as for example those described in British patent No. 1,234,467, in European patent applications No. 127,882 and No. 158,340 and in the British patent application No. 2,167,036 all in the name of Tanabe Seiyaku Co. Ltd.

Most of these methods substantially foresee the following reaction scheme. 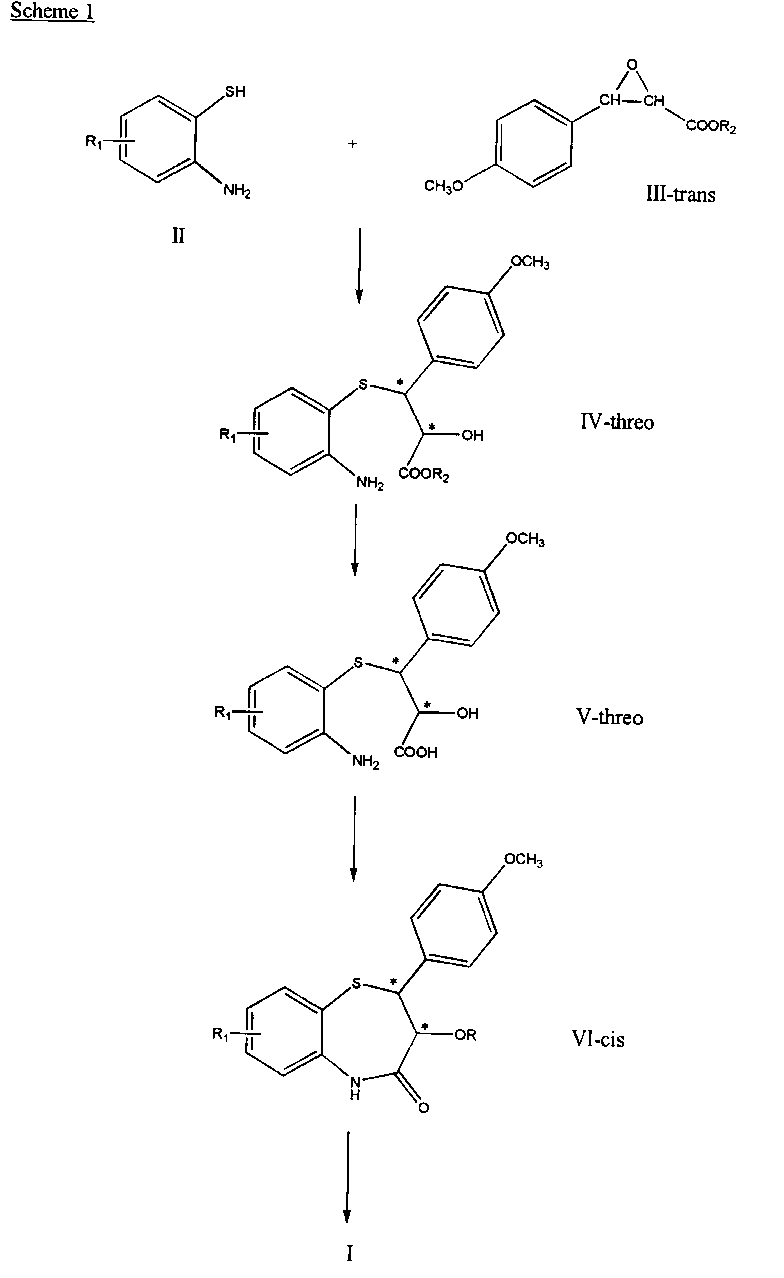 wherein R₁ represents a hydrogen or chlorine atom, R₂ represents a lower alkyl and the asterisks mark the asymmetric carbon atoms.

EP-A-378455 is a relevant prior art only as far as Art. 54(3) EPC is concerned.

The cyclization of methyl (2S,3S)-2-hydroxy-3-(2-aminophenylthio)-3-(4-methylphenyl)-propionate which can bear a chlorine substituent on the 2-aminophenylthio moiety by heating in the presence of a chlorinated solvent having a boiling point higher than 70°C and of methanesulfonic acid is described therein.

Chem. Pharm. Bull., vol 18, No. 10, 1970, pages 2028-2037 generally describes the cyclization of esters of formula 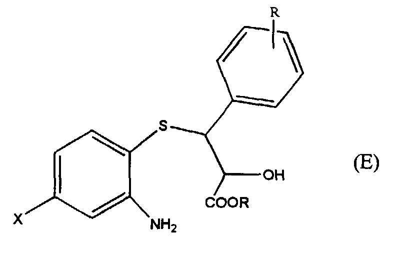 by heating in the presence of sulfuric or acetic acid.

This paper clearly states that the cyclization of the corresponding carboxylic acids gives better results than the cyclization of the esters of formula (E).

The only example reported in detail in the Chem. Pharm. Bull. article concerns the cyclization of an ester unsubstituted on the phenyl ring adjacent to the C-OH group by heating in 20% sulphuric acid or in acetic acid without other solvents.

Object of the present invention is a process for the preparation of compounds of formula 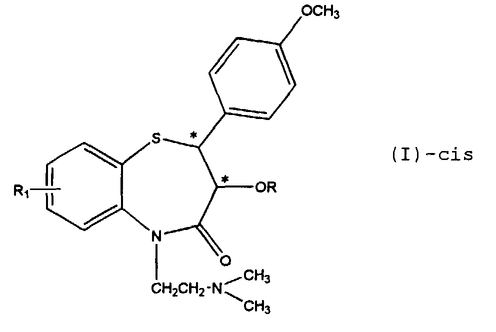 wherein
R represents a hydrogen atom or acetyl,
R₁ represents a hydrogen atom or chlorine atom,
   the asterisks mark the asymmetric carbon atoms;
   which comprises the cyclization of the optically active amino ester of formula 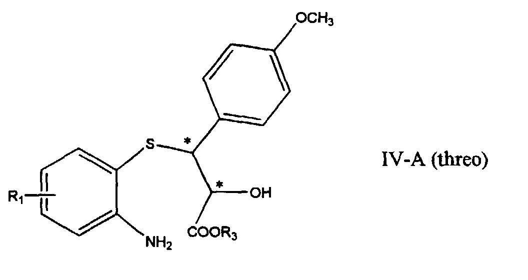 wherein
R₁ represents a hydrogen atom or chlorine atom,
R₃ represents a C₁-C₃ alkyl,
the asterisks mark the asymmetric carbon atoms;
   by heating preferably in the presence of a solvent such as xylene or toluene and of small amounts of an acid, e.g. p.toluenesulfonic acid, sulfuric acid in water being excluded and the cyclization of methyl (2S,3S)-2-hydroxy-3-(2-aminophenylthio)-3-(4-methoxyphenyl)-propionate which can bear a chlorine substituent on the 2-aminophenylthio moiety by heating in the presence of a chlorinated solvent having a boiling point higher than 70°C and methanesulfonic acid being excluded too.

The process object of the present invention represents an extremely advantageous process, simple and unexpensive, for the preparation of the compound of formula 1.

By a practical point of view, the process object of the invention allows, in fact, to avoid the subsequent hydrolysis of the amino ester IV, reported in scheme 1, by carrying out the cyclization directly on the amino ester.

With the aim to better illustrate the present invention the following example is given,

### Example

### Cyclization of methyl (2S,3S)-2-hydroxy-3-(2-aminophenylthio)-3-(4-methoxyphenyl)-pronionate

To a suspension of methyl (2S,3S)-2-hydroxy-3-(2-aminophenylthio)-3-(4-methoxyphenyl)-propionate (19.4 g; 0.058 moles) in xylene (223 ml), monohydrate p.toluenesulfonic acid (1.1 g) was added.

The mixture was heated to reflux under stirring for 5 hours. Then it was cooled and the insoluble was filtered and dried in an oven under vacuum at 70°C to give (2S,3S)-2,3-dihydro-3-hydroxy-2-(4-methoxyphenyl)-1,5-benzothiazepin-4(5H)-one (16.1 g - 90% yield) with titre 98% weight/weight.

## Claims

1. A process for the preparation of compounds of formula 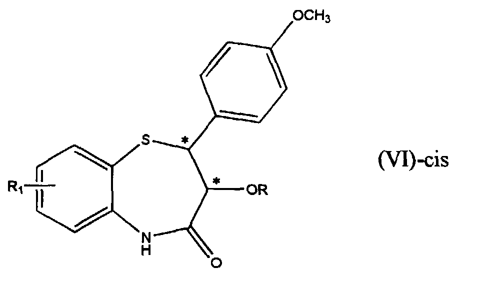 wherein
R represents a hydrogen atom or acetyl,
R₁ represents a hydrogen atom or chlorine atom,
the asterisk mark the asymmetric carbon atoms;
which comprises the cyclization of the optically active amino ester of formula 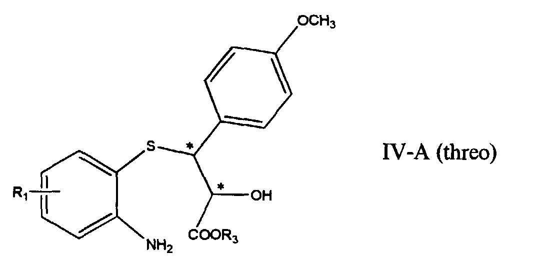 wherein
R₁ represents a hydrogen atom or chlorine atom,
R₃ represents a C₁-C₃ alkyl,
the asterisk mark the asymmetric carbon atoms;
by heating in the presence of a solvent and of an acid, sulfuric acid in water being excluded and the cyclization of methyl (2S,3S)-2-hydroxy-3-(2-aminophenylthio)-3-(4-methoxyphenyl)propionate which can bear a chlorine substituent on the 2-aminophenylthio moiety by heating in the presence of a chlorinated solvent having a boiling point higher than 70°C and of methanesulfonic acid being excluded too.

2. A process according to claim 1 wherein the solvent is xylene or toluene.

3. A process according to claim 1 wherein the acid is present in a molar amount of about 10%.

4. A process according to claim 1 wherein the acid is p.toluenesulphonic acid.

5. A process for the preparation of compounds of formula 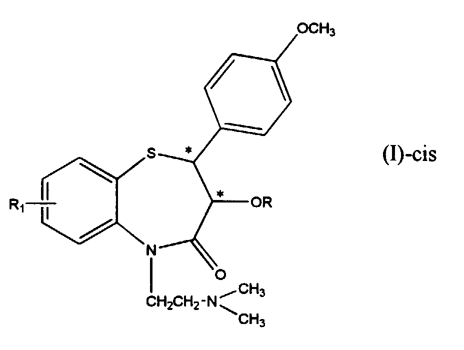 wherein
R represents a hydrogen atom or acetyl,
R₁ represents a hydrogen atom or chlorine atom,
the asterisks mark the asymmetric carbon atoms;
which comprises the cyclization according to claim 1 to afford the compound of formula (VI-cis) and the further alkylation and optional acetylation.

## Patentansprüche

1. Ein Verfahren zur Herstellung von Verbindungen der Formel 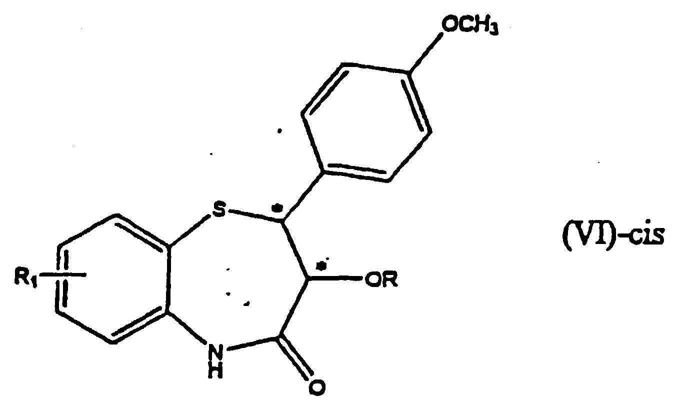 in der
R ein Wasserstoffatom oder Acetyl bedeutet,
R₁ ein Wasserstoffatom oder Chloratom bedeutet,
der Stern (*) die asymmetrischen Kohlenstoffatome kennzeichnet;
das die Zyklisierung des optisch aktiven Aminoesters der Formel 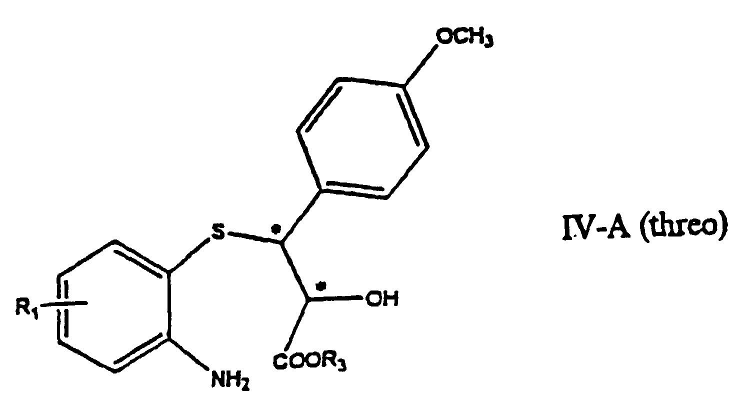 umfaßt, in der
R₁ ein Wasserstoffatom oder Chloratom bedeutet,
R₃ ein C₁-C₃-Alkyl bedeutet,
der Stern (*) die asymmetrischen Kohlenstoffatome kennzeichnet;
durch Erhitzen in Gegenwart eines Lösungsmittels und einer Säure, wobei Schwefelsäure in Wasser ausgeschlossen ist und die Zyklisierung von Methyl(2S,3S)-2-hydroxy-3-(2-aminophenylthio)-3-(4-methoxyphenyl)propionat, das einen Chlorsubstituent an dem 2-Aminophenylthiorest tragen kann, durch Erhitzen in Anwesenheit eines chlorierten Lösungsmittels mit einem Siedepunkt über 70°C und von Methansulfonsäure auch ausgeschlossen ist.

2. Ein Verfahren nach Anspruch 1, wobei das Lösungsmittel Xylol oder Toluol ist.

3. Ein Verfahren nach Anspruch 1, wobei die Säure in einer molaren Menge von etwa 10 % vorliegt.

4. Ein Verfahren nach Anspruch 1, wobei die Säure p-Toluolsulfonsäure ist.

5. Ein Verfahren zur Herstellung einer Verbindung der Formel 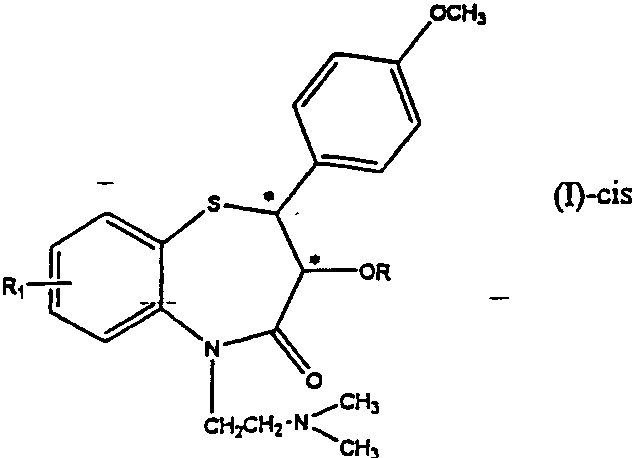 in der
R ein Wasserstoffatom oder Acetyl bedeutet,
R₁ ein Wasserstoffatom oder Chloratom bedeutet,
der Stern (*) die asymmetrischen Kohlenstoffatome kennzeichnet;
das die Zyklisierung gemäß Anspruch 1 zur Erzielung der Verbindung der Formel (VI-cis) und die weitere Alkylierung und gegebenenfalls Acetylierung umfaßt.

## Revendications

1. Procédé de préparation de composés de formule 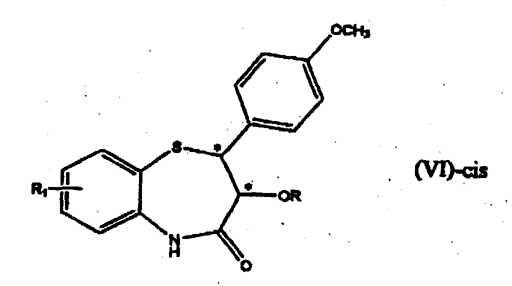 dans laquelle
R représente un atome d'hydrogène ou un groupement acétyle,
R₁ représente un atome d'hydrogène ou de chlore,
l'astérisque marque les atomes de carbone asymétriques :
comprenant la cyclisation de l'amino-ester optiquement actif de formule 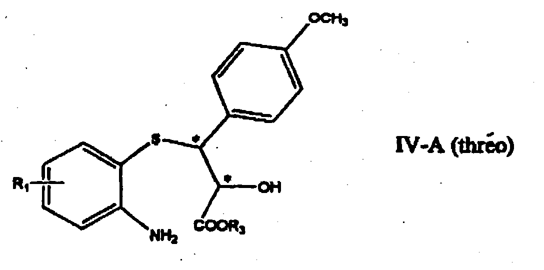 dans laquelle
R₁ représente un atome d'hydrogène ou de chlore,
R₃ représente un groupement alkyle en C₁-C₃,
l'astérisque marque les atomes de carbone asymétriques :
par chauffage en présence d'un solvant et d'un acide à l'exclusion de l'acide sulfurique dans l'eau, et la cyclisation du (2S,3S)-2-hydroxy-3-(2-aminophénylthio)-3-(4-méthoxyphényl)propionate de méthyle pouvant porter un substituant chlore sur la fraction 2-aminophénylthio par chauffage en présence d'un solvant chloré ayant un point d'ébullition supérieur à 70°C et d'acide méthanesulfonique étant également exclue.

2. Procédé selon la revendication 1, dans lequel le solvant est le xylène ou le toluène.

3. Procédé selon la revendication 1, dans lequel l'acide est présent en une quantité molaire d'environ 10%.

4. Procédé selon la revendication 1, dans lequel l'acide est l'acide p-toluènesulfonique.

5. Procédé pour la préparation de composés de formule 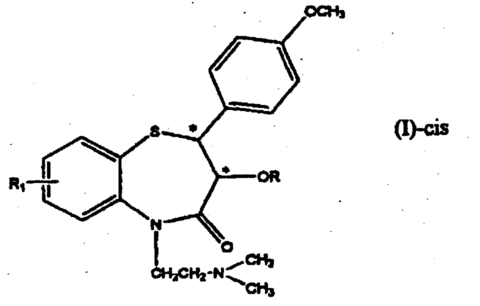 dans laquelle
R représente un atome d'hydrogène ou un groupement acétyle,
R₁ représente un atome d'hydrogène ou de chlore,
l'astérisque marque les atomes de carbone asymétriques :
comprenant la cyclisation selon la revendication 1 pour donner le composé de formule (VI-cis) suivie d'une alkylation et d'une éventuelle acétylation.
